# FASCICULE DE BREVET EUROPEEN

(11) **EP 1 802 352 B1**
(45) Date de publication et mention de la délivrance du brevet: **27.03.2013**
(21) Numéro de dépôt: 05807538.3
(22) Date de dépôt: 30.09.2005
(51) Int. Cl.: A61K 49/14, A61K 51/08, A61P 9/10

(54) **UTILISATION DE LIGANDS DE VCAM-1 POUR LA DETECTION ET/OU LE TRAITEMENT DE MALADIES CARDIOVASCULAIRES**
VERWENDUNG VON VCAM-1-LIGANDEN ZUR ERKENNUNG UND/ODER BEHANDLUNG KARDIOVASKULÄRER KRANKHEITEN
USE OF VCAM-1 LIGANDS FOR DETECTING AND/OR TREATING CARDIOVASCULAR DISEASES

(30) Priorité: 01.10.2004 FR 0410420
(43) Date de publication de la demande: 04.07.2007
(73) Titulaire: UNIVERSITE JOSEPH FOURIER, F-38041 Grenoble Cédex (FR)
(72) Inventeur: BOTURYN, Didier, F-38500 La Buisse (FR); RIOU, Laurent, F-38100 Grenoble (FR); GHEZZI, Catherine, F-38000 Grenoble (FR); FAGRET, Daniel, F-38000 Grenoble (FR); DUMY, Pascal, F-38580 Allevard (FR)
(74) Mandataire: Colombet, Alain André
(86) Numéro de dépôt international: PCT/FR2005/002423
(87) Numéro de publication internationale: WO 2006/037882

(56) Documents cités:
- HAMILTON ET AL.: "Intravascular ultrasound molecular imaging of atheroma components in vivo" JOURNAL OF THE AMERICAN COLLEGE OF CARDIOLOGY, vol. 43, no. 3, 4 février 2004 (2004-02-04), pages 453-460, XP001206522
- HAMILTON ET AL.: "Intravascular ultrasound molecular imagign of ECAM-1, VCAM-1, fibrinogen and fibrin in a yucatan miniswine model of atherosclerosis" JOURNAL OF THE AMERICAN COLLEGE OF CARDIOLOGY, vol. 41, no. 6, 19 mars 2003 (2003-03-19), page 300A, XP001206523
- XIN YU ET AL.: "high-resolution mRI characterization of human thrombus using a novel fibrin-targeted paramagnetic nanoparticle contrast agent" MAGNETIC RESONANCE IN MEDICINE, vol. 44, 2000, pages 867-872, XP002328224
- FAYAD ET AL.: "clinical imaging of the high-risk or vulnerable atherosclerotic plaque" CIRC. RES., vol. 89, 2001, pages 305-316, XP002328223 cité dans la demande
- KRAAN ET AL.: "Measurement of cytokine and adhesion molecule expression in synovial tissue by dgital image analysis" ANNALS OF RHEUMAT. DISEASES, vol. 60, 2001, pages 296-298, XP002328222

### LISTAGE DE SEQUENCES

<110> INSERM
<120> Utilisation de ligands de VCAM-1 pour la détection et/ou le traitement de
   maladies cardiovasculaires
<130> BET 04P0419
<160> 4
<170> PatentIn version 3.1
<210> 1
   <211> 10
   <212> PRT
   <213> Séquence Artificielle
<220>
   <223> peptide
<400> 1
   Tyr Arg Leu Ala Ile Arg Leu Asn Glu Arg
   1               5                   10
<210> 2
   <211> 20
   <212> PRT
   <213> Séquence Artificielle
<220>
   <223> peptide
<400> 2
   Tyr Arg Leu Ala Ile Arg Leu Asn Glu Arg Arg Glu Asn Leu Arg Ile
   1               5                   10                  15
   Ala Leu Arg Tyr
               20
<210> 3
   <211> 12
   <212> PRT
   <213> Séquence Artificielle
<220>
   <223> peptide
<400> 3
   Tyr Arg Leu Ala Ile Arg Leu Asn Glu Arg Gly His
   1               5                   10
<210> 4
   <211> 10
   <212> PRT
   <213> Séquence Artificielle
<220>
   <223> peptide circulaire
<400> 4
   Gly Lys Ala Lys Pro Gly Lys Lys Lys Pro
   1               5                   10

## Description

L'invention est relative à l'utilisation de ligands de VCAM-1 en imagerie médicale, notamment pour la caractérisation et/ou le suivi thérapeutique de maladies cardiovasculaires et plus particulièrement pour la détection de plaque d'athérome coronaire vulnérable. L'invention concerne également l'utilisation de ligands de VCAM-1 pour la fabrication d'un médicament destiné au traitement d'une maladie cardiovasculaire.

Les maladies cardiovasculaires, et notamment l'infarctus du myocarde, représentent la première cause de mortalité dans les pays industrialisés. L'athérosclérose est le principal facteur causal des maladies coronaires.

Le processus conduisant au développement d'une plaque d'athérome implique une infiltration lipidique initiale de l'intima d'une artère, marquée par la pénétration passive et l'accumulation des lipoprotéines de basse densité (LDL-Cholestérol) suivie d'une modification oxydative de ces LDL. Ce mécanisme favorise l'adhésion des monocytes circulant au niveau de la surface de l'endothélium. Ces monocytes pénètrent l'espace sous endothélial et se transforment en macrophages qui entraînent ensuite une réaction inflammatoire chronique locale et la production de cytokines pro-inflammatoires.

La plaque d'athérome comprend un coeur lipidique et une chape fibreuse, constituée de cellules musculaires lisses, de collagènes et d'une matrice extra-cellulaire, et qui isole le coeur lipidique de la lumière artérielle. La plaque, lorsqu'elle évolue et fait protrusion dans la lumière artérielle, entraîne la formation d'une sténose artérielle qui peut induire une ischémie.

Le risque principal est la rupture de la plaque qui survient au niveau de la chape fibreuse et met en contact le sang avec les éléments thrombogènes du coeur lipidique, exposant ainsi le sujet à une thrombose artérielle. En particulier, la rupture d'une plaque d'athérome coronaire vulnérable est à l'origine de la plupart des évènements coronariens (infarctus du myocarde, mort subite, angor instable). La vulnérabilité de la plaque, c'est-à-dire sa propension à la rupture, résulte essentiellement de l'importance du coeur lipidique et/ou d'une fragilisation de la chape fibreuse, par exemple par des mécanismes inflammatoires.

La plaque d'athérome coronaire vulnérable, qui est caractérisée par un coeur lipidique mou important, une fine chape fibreuse et une inflammation exacerbée, est invisible à la coronarographie mais est parfaitement étudiée de maniére invasive par l'imagerie ultrasonore intra-coronaire. Cependant, aucune méthode d'imagerie atraumatique ne permet actuellement de mettre en évidence ces plaques d'athérome coronaires vulnérables.

L'imagerie par Résonance Magnétique est une technique non invasive qui a été utilisée pour l'imagerie de plaques carotidiennes. Cependant, sa résolution ne permet pas l'imagerie des plaques d'athérome coronaires. Une autre technique non invasive est la Médecine Nucléaire: elle permet par exemple la réalisation d'images scintigraphiques après injection de traceurs radioactifs spécifiques d'une cible biologique. Cependant, actuellement, il n'existe aucun traceur radioactif utilisable en Médecine Nucléaire pour la détection des plaques vulnérables. De nombreuses cibles ont été étudiées (LDL-Tcm^{99m}, LDL-iode¹²³, Anticorps anti-récepteur Fc des macrophages, etc...) mais aucune n'est suffisamment spécifique des plaques d'athérome vulnérables (Narula et al., 1999 ; Fayad et Fuster, 2001).

Les articles Hamilton et al. (2004) J. Am. Coll Cardio. 43:453-460 et Hamilton et al. (2003) J. Am. Coll. Cardio. 41:300A décrivent l'utilisation d'immunoliposomes anti-VCAM-1, utilisés comme agent de contraste dans une technique de détection de lésions athérosclérotiques par ultrasons intravasculaires, *i.e.* une technique d'imagerie invasive.

L'article Yu et al. (2000) Magnetic Resonance in Medicine 44:867-872 décrit l'utilisation, *in vitro,* de nanoparticules paramagnétiques spécifiques de la fibrine comme agent de contraste pour détecter les constituants de plaques athérosclérotiques humaines par IRM.

L'article Kraan et al. (2001) Annals of Rheumat. Diseases 60296-298 décrit des anticorps monoclonaux anti-VCAM-1 utilisés comme agents de contraste *in vitro,* en combinaison avec des colorants.

Or le développement de traceurs radioactifs spécifiques de la plaque d'athérome coronaire vulnérable devrait permettre de déceler simplement, par détection externe de la radioactivité, la présence et le nombre de plaques vulnérables. Une telle détection est un enjeu majeur pour prédire la survenue d'accidents coronariens chez un patient et pour pouvoir adapter la thérapie en fonction de risque d'accidents coronariens chez ce patient.

L'existence d'un phénomène inflammatoire est une caractéristique de la plaque vulnérable. Il a notamment été montré que VCAM-1, une molécule d'adhésion endothéliale impliquée dans l'adhésion des monocytes à l'endothélium vasculaire, est surexprimée à la surface des plaques d'athérome coronaire vulnérables. Toutefois VCAM-1 n'a jamais été choisi comme cible pour le développement d'un traceur radioactif de la plaque d'athérome vulnérable.

Les inventeurs ont démontré que des ligands de VCAM-1 radioactifs constituent des traceurs spécifiques de la plaque d'athérome coronaire vulnérable et permettent son imagerie par simple détection externe de la radioactivité après injection intraveineuse.

Des traceurs ont en particulier été développés sur la base du peptide B2702 constitué des résidus 75 à 84 de la molécule HLA-B2702. Ce peptide (B2702.84-75) ainsi que le peptide constitué du motif 75-84 répété (B2702.84-75/75-84) se lient spécifiquement à VCAM-1 comme montré par Ling et al. (2000).

Après synthèse et marquage radioactif, les traceurs ont été évalués sur des modèles animaux utilisés pour l'étude du développement de l'athérosclérose. Les résultats des inventeurs montrent que, après injection *in vivo,* les ligands de VCAM-1 marqués se fixent spécifiquement sur les plaques d'athérome se développant sur les aortes de lapins hyperlipidémiques.

Ces résultats indiquent donc que des agents de contraste contenant un ligand de VCAM-1 sont utiles en imagerie médicale, en particulier pour la détection d'une maladie cardiovasculaire chez un patient. La capacité de détecter et surveiller la formation d'une plaque d'athérosclérose, plus spécifiquement d'une plaque d'athérome coronaire vulnérable, est un enjeu majeur compte tenu du rôle de ces plaques dans la survenue de syndromes ischémiques aigus. L'utilisation d'agents de contraste telle que décrite dans la demande est donc particulièrement utile pour la détection des plaques vulnérables chez une population présentant un risque cardiovasculaire. En outre ces agents de contraste peuvent être utilisés pour établir un index pronostique permettant d'adapter un traitement thérapeutique aux risques du patient.

### Définitions

"VCAM-1" (Vascular Cell Adhesion Molecule 1) désigne une protéine d'adhésion cellulaire dont la transcription est induite dans les cellules endothéliales mais qui est également exprimée par d'autres types cellulaires. VCAM-1 a été découverte et clonée par Osbom et al. en 1989. VCAM-1 interagit avec l'intégrine α4β1, aussi appelée VLA-4 (Very Late Antigen 4), qui est exprimée de manière constitutive par les lymphocytes et les monocytes, notamment. Comme d'autres molécules d'adhésion, telles que ICAM-1, 2, et 3, VCAM-1 est impliquée dans l'adhésion des monocytes à l'endothélium au cours de l'athérosclérose. VCAM-1 interagit également avec l'intégrine α4β7 pour le recrutement de lymphocytes au niveau de l'intestin.

Par "ligand de VCAM-1" on entend une molécule capable de se lier de manière sélective à VCAM-1. Préférentiellement le ligand est spécifique de VCAM-1, c'est-à-dire qu'il se lie à VCAM-1 à l'exclusion de toute autre molécule. Un ligand peut être une petite molécule, telle qu'un composé chimique, ou une grand molécule telle que des polypeptides ou protéines, y compris des anticorps, des facteurs de croissance, ou des intégrines. Des exemples de ligands de nature polypeptidiques de VCAM-1 incluent les intégrines α4β1 et α4β7, ou encore les peptides B2702.84-75 et B2702.84-75/75-84, ou le composé 4(B 2702.84-75)Raft. Les ligand de VCAM-1 incluent également des aptamères.

Les "aptamères" constituent une classe de molécules qui représentent une alternative aux anticorps en terme de reconnaissance moléculaire. Les aptamères sont des séquences d'oligonucléotides ayant la capacité de reconnaître virtuellement n'importe quelle classe de molécules cibles avec une haute affinité et spécificité. De tels ligands peuvent être isolés par un criblage appelé SELEX (Systematic Evolution of Ligands by EXponential enrichment) d'une banque de séquences aléatoires, comme décrit dans Tuerk et Gold (1990). La banque de séquence aléatoire peut être obtenue par synthèse d'ADN par chimie combinatoire.Dans une telle banque, chaque membre est un oligomère linéaire, éventuellement chimiquement modifié, correspondant à une séquence unique. Les possibles modifications, applications et avantages de cette classe de molécules ont fait l'objet d'une revue par Jayasena (1999). Les aptamères peuvent également être de nature peptidique. Ils consistent en une région variable conformationnellement contrainte, assemblée sur une plateforme protéique, telle que la Thioredoxin A de E. coli, et peuvent être sélectionnés à partir de banques combinatoires par des méthodes de double hybride (Colas et al., 1996).

Le peptide "B2702.84-75" désigne le peptide de séquence YRLAIRLNER (SEQ ID n°1), qui est constitué par la séquence inversée des 10 résidus C-terminaux (résidus 84 à 75) de l'hélice alpha α1 de la molécule HLA-B2702 (Ling et al., 2000).

Le peptide "B2702.84-75/75-84" désigne le peptide de séquence YRLAIRLNERRENLRIALRY (SEQ ID n°2), constitué par un dimère inversé des 10 résidus C-terminaux de l'hélice alpha α1 de la molécule HLA-B2702 (résidus 84-75/75-84) (Ling et al., 2000).

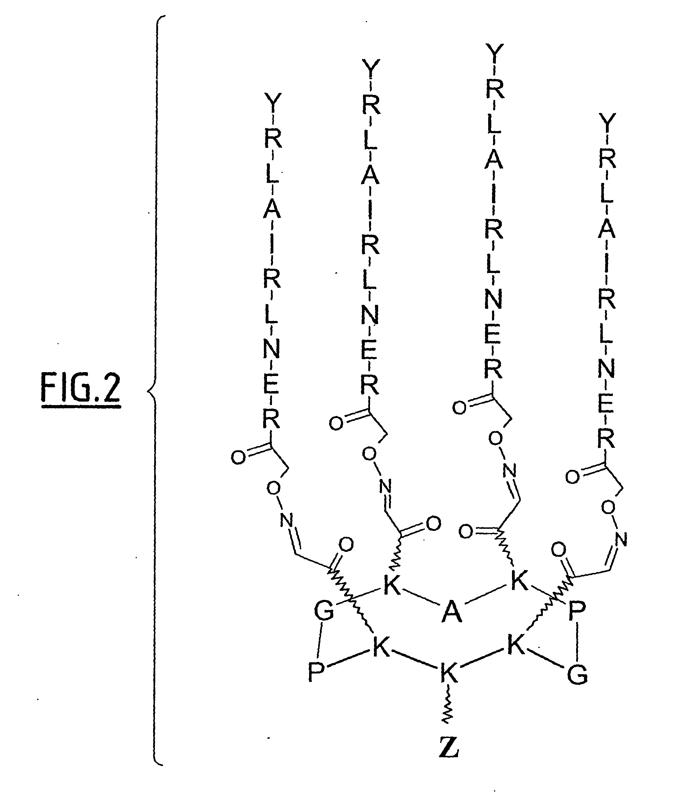

Le composé 4(B 2702.84-75)Raft désigne une molécule constituée par une plateforme cyclopeptidique ₁GKAKPGKKKP₁₀ ("Raft") portant sur une même face quatre peptides B 2702.84-75 greffés sur les lysines en position 2, 4, 7 et 9 du décapeptide. La plateforme cyclopeptidique porte sur son autre face un groupement Z, greffé via la lysine en position 8, et désigne un agent de contraste quelconque permettant l'imagerie de la liaison entre VCAM-1 et le composé 4(B 2702.84-75)Raft. La structure du composé 4(B 2702.84-75)Raft est montrée en figure 2. La synthèse et le greffage de la plateforme cyclopeptidique peut être effectuée conformément au procédé décrit dans la demande internationale WO 2004026894.

Par [^{99m}Tc]-B 2702.84-75/75-84, [^{99m}Tc]-B 2702.84-75 ou [^{99m}Tc]-4(B 2702.84-75)Raft on désigne un peptide B 2702.84-75/75-84, B 2702.84-75 ou 4(B 2702.84-75)Raft, marqué par le technétium 99 et dont la séquence peptidique a éventuellement été modifiée, pour les besoins du marquage, par ajout d'un, deux ou plus, acide(s) aminé(s), en particulier par ajout au moins d'une histidine du côté C-terminal ou N-terminal de la séquence peptidique. Par exemple, le peptide [^{99m}Tc]-B 2702.84-75 désigne de préférence le peptide de séquence YRLAIRLNERGH (SEQ ID n°3) dans lequel le technétium 99 est lié à l'histidine.

Dans le contexte de la présente invention, on désigne par "maladie cardiovasculaire" une maladie une lésion, ou un symptôme lié à une processus d'athérogenèse affectant le système cardiovasculaire. Cela inclut notamment les états marquant le développement d'une plaque athéromateuse (les plaques sont classifiées en stades de progression I à VI, selon la classification internationale de Stary), ainsi que les complications découlant de la formation d'une plaque athéromateuse (sténose, ischémie) et/ou de son évolution vers un accident ischémique aigu (thrombose, embolie, infarctus, rupture artérielle). Les maladies cardiovasculaires désignent par exemple l'athérosclérose, une plaque d'athérome, en particulier la plaque vulnérable, la maladie coronarienne, l'angor, la thrombose, l'accident vasculaire cérébral, l'infarctus du myocarde, la sténose vasculaire, l'infarctus.

La "maladie coronarienne" est la manifestation la plus courante de la maladie cardiovasculaire. Il s'agit d'une maladie progressive, due à une mauvaise irrigation du muscle cardiaque, consécutive au rétrécissement (sténose) ou à la calcification (sclérose) d'une ou des artères coronaires. Le symptôme principal de la maladie coronarienne se manifeste sous la forme de douleurs qui constituent un angor (stable ou instable), aussi appelé angine de poitrine. L'obstruction complète d'une ou des artères coronaires conduit à l'infarctus.

L"'infarctus" désigne un foyer circonscrit de nécrose dû à une obstruction artérielle. Plus spécifiquement, l'infarctus du myocarde est une nécrose du myocarde qui résulte généralement d'une thrombose coronaire aiguë, secondaire à une rupture de plaque (généralement une plaque instable ou plaque vulnérable) entraînant l'agrégation plaquettaire puis l'occlusion coronaire.

La "thrombose" correspond à la coagulation du sang dans les cavités vasculaires (artères, veines, capillaires ou cavités cardiaques) conduisant à la formation d'un thrombus.

L"'embolie" est la migration intravasculaire d'un corps étranger, le plus souvent constitué par un caillot sanguin (thombus), et son arrêt brusque dans un vaisseau dont le calibre est insuffisant pour le laisser passer. Les conséquences locales de l'embolie sont des perturbations circulatoires liées à l'obstruction vasculaire, conduisant le plus souvent à un infarctus.

L"'ischémie" désigne la diminution de l'apport sanguin artériel dans un territoire de l'organisme. Ses principales causes locales sont la thrombose et l'embolie.

--L'expression-"plaque-vulnérabie"-désigne-une-plaque-d'-athérome-présentant une chape fibreuse fine (environ 65 à 150 µm d'épaisseur) et un coeur lipidique important. Ces plaques instables, qui présentent une tendance à la rupture, se rencontrent au niveau des artères coronaires et au niveau de l'aorte et de ses branches. La rupture des plaques coronariennes vulnérable provoque des "syndromes coronariens aigus" : en cas de thrombose occlusive complète, il s'agit de l'infarctus du myocarde ; lorsque la thrombose de l'artère reste incomplète il s'agit de l'angor instable. Au niveau de la carotide, les plaques vulnérables sont plus sténotiques et moins inflammatoires. Elles expriment également VCAM-1.

Un "agent de contraste" désigne une substance ou une composition qui, administrée dans l'organisme, permet de marquer de manière détectable des organes ou des structures (tissu, cellule, récepteur) qui, sans agent de contraste, sont peu ou non visibles en imagerie médicale. Par extension, l'expression "agent de contraste" est utilisée pour désigner un traceur associé à un marqueur.

Par "traceur", on entend de manière générale une substance qui peut se localiser de manière sélective au niveau d'une structure particulière de l'organisme (tissu, organe, cellule, récepteur, par exemple). Il peut s'agir d'un élément simple (un atome), d'une molécule (une protéine par exemple), ou d'une structure plus complexe (liposome, cellule, etc... ). Dans le cadre de la présente demande le traceur est un ligand de VCAM-1.

Par "marqueur" on entend un composé qui produit un signal détectable. Lorsqu'il est associé à un traceur, il permet de suivre le devenir du traceur dans l'organisme. Le marqueur peut être un agent de contraste IRM, un agent de contraste en scintigraphie, un agent de contraste en imagerie aux rayons X, un agent de contraste ultrason, un agent de contraste en imagerie optique.

### Agent de contraste

Les inventeurs ont démontré que des ligands de VCAM-1 constituent des traceurs spécifiques de la plaque d'athérome, en particulier de la plaque aortique et/ou coronarienne vulnérable, et permettent sa détection par imagerie.

La demande décrit donc l'utilisation d'un ligand de VCAM-1 pour la fabrication d'un agent de contraste utile pour l'imagerie médicale. Toutefois, la technique d'imagerie médicale susceptible d'être utilisée est de préférence une technique d'imagerie *in vivo,* non invasive.

Préférentiellement, le ligand de VCAM-1 est sélectionné dans le groupe constitué du peptide B 2702.84-75/75-84, du peptide B 2702.84-75, du composé 4(B 2702.84-75)Raft, et d'aptamères ligands de VCAM.

La présente invention concerne ainsi l'utilisation d'un ligand de VCAM-1 comme agent de contraste dans l'imagerie médicale *in vivo,* non invasive, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé.

Le ligand de VCAM-1 peut également être un dérivé des peptides B 2702.84-75 ou B 2702.84-75/75-84, ou un dérivé du composé 4(B 2702.84-75)Raft. Par "dérivé" de ces peptides ou de ce composé, on entend un peptide ou composé dans lequel la séquence RENLRIALRY constituant la séquence 84-75 du peptide B 2702 a été modifiée par ajout, suppression, substitution ou modification d'au moins un acide aminé. La séquence RENLRIALRY peut en particulier être modifiée par substitution d'un, deux ou trois acides aminés. La substitution peut être conservative ou non. La substitution est conservative lorsqu'un acide aminé est substitué par un acide aminé ayant des propriétés similaires (par exemple, polarité, potentiel de liaison d'hydrogène, acidité, basicité, hydrophobicité, présence d'un groupe aromatique, etc...). Des acides aminés ayant des propriétés similaires sont bien connus de l'homme du métier, par exemple, arginine, histidine et lysine sont des acides aminés hydrophiles-basiques qui peuvent être interchangeables ; l'isoleucine, un acide aminé hydrophobe, peut être remplacé par la leucine, la méthionine ou la valine. Un acide aminé naturel peut être remplacé par un acide aminé non naturel, tel qu'un acide aminé en configuration D, acide aminé bêta ou gamma. La séquence RENLRIALRY peut être modifiée en remplaçant une ou plusieurs liaisons amide par une liaison conférant une stabilité accrue in vivo, par exemple conférant une résistance accrue à la protéolyse.

Un dérivé du composé 4(B 2702.84-75)Raft peut présenter un, deux, trois ou quatre peptides B 2702.84-75 dérivés, lesdits peptides B 2702.84-75 dérivés greffés sur la plateforme cyclique pouvant être modifiés de manière identique ou non.

Les peptides dérivé B 2702.84-75 ou dérivé B 2702.84-75/75-84, ou le composé dérivé 4(B 2702.84-75)Raft conservent la capacité de lier VCMA1. Préférentiellement, ces dérivés présentent une affinité de liaison avec VCAM-1 égale ou accrue par rapport au peptide ou composé correspondant, B 2702.84-75, B 2702.84-75/75-84, ou 4(B 2702.84-75)Raft. La mesure de l'affinité de liaison d'un ligand avec VCAM-1 peut être effectuée par exemple en utilisant la technique de polarisation de fluorescence (Checovich et al., 1995).

Le principe de la polarisation de fluorescence repose sur l'étude de la polarisation de la lumière émise par un composé marqué avec un fluorophore (tel que la fluorescéine par exemple). Lorsqu'il est en solution, un composé fluorescent est en rotation avec une vitesse constante, qui est dépendante de la masse du ligand et de la viscosité du milieu. Ce composé a la possibilité, lorsqu'on le soumet à une excitation lumineuse, de renvoyer une lumière dépolarisée, et ce dans toutes les directions de l'espace. La lumière renvoyée est analysée grâce à deux capteurs fixes séparés par un angle de 90°. Ces capteurs permettent de recueillir la lumière réémise dans un plan de réception et d'exprimer deux grandeurs (l'anisotropie et la polarisation) à partir des intensités recueillies issues des faisceaux parallèles ou perpendiculaires au faisceau d'excitation. L'anisotropie et la polarisation sont deux facteurs liés l'un à l'autre. Si on ajoute un ligand à la solution contenant un composé fluorescent, les propriétés lumineuses du composé fluorescent changent dans le complexe composé/ligand et la lumière réémise devient polarisée. L'anisotropie représentée en fonction de la concentration en ligand en solution évolue selon une courbe sigmoïde, avec une phase d'augmentation progressive de l'intensité relative de fluorescence liée à l'ajout croissant de ligand et donc à l'apparition de complexe (composé/ligand), suivie d'un plateau haut reflétant la présence de complexe qui polarise la lumière en raison d'une vitesse de rotation en solution ralentie par rapport au composé fluorescent seul. Les mesures de polarisation de fluorescence peuvent être effectuées par exemple avec un spectromètre de luminescence PERKIN ELMER LS50. Pour la mesure de l'affinité de ligands de VCAM-1 le composé marqué par le fluorophore peut être indistinctement le ligand de VCAM-1 ou VCAM-1. On peut alors comparer les affinités de différents ligands de VCAM-1 pour VCAM-1 en déterminant graphiquement la concentration en ligand (si VCAM-1 est le composé fluorescent marqué) nécessaire pour former 50% des complexes VCAM-1/ligand de VCAM-1.

L'utilisation d'un ligand de VCAM-1 pour la fabrication d'un agent de contraste est plus particulièrement pour le diagnostic par imagerie médicale d'une maladie cardiovasculaire.

Les techniques d'imagerie médicale utilisées pour diagnostiquer une maladie cardiovasculaire, et en particulier déceler les plaques d'athérosclérose, regroupent des techniques invasives telles que l'angiographie ou la coronarographie, l'échographie endocoronaire, ou des techniques non invasives telles que la vélocimétrie doppler, l'angiographie par imagerie par résonance magnétique ou la médecine nucléaire. Les techniques de médecine nucléaire plus particulièrement employée dans le domaine cardio-vasculaire incluent la scintigraphie de perfusion.

L'utilisation d'un agent de contraste, choisi en fonction de la technique d'imagerie utilisée (agent de contraste IRM, agent de contraste en scintigraphie, agent de contraste en imagerie aux rayons X, agent de contraste ultrason, agent de contraste en imagerie optique), permet de cibler de manière spécifique un tissu, un organe, ou une zone pathologique.

La scintigraphie, par exemple, repose sur l'administration (généralement par voie intra-vasculaire) d'un agent de contraste, aussi appelé radio-pharmaceutique, constitué d'un traceur marqué par un isotope radioactif. La localisation spécifique de cet agent de contraste dans l'organisme est ensuite déterminée par détection des rayons gamma ou bêta émis.

Les ligands de VCAM-1 peuvent être utilisés avec différentes techniques d'imagerie. A titre d'exemples des techniques d'imagerie et des marqueurs associés qui peuvent être utilisés incluent l'imagerie TEP, le marqueur étant de préférence du Fluor 18 (¹⁸F) ; l'IRM, les marqueurs étant de préférence des nanoparticules d'oxyde de fer ou du Gadolinium ; l'imagerie par fluorescence, le marqueur étant de préférence la Fluorescine, Alexa ou Cyanine ; l'imagerie par chemiluminescence, le marqueur étant de préférence le luminol ; l'imagerie par bioluminescence, le marqueur étant la luciférase ou la phosphatase alkaline notamment. L'imagerie optique regroupe les techniques d'imagerie par fluorescence, d'imagerie par chemiluminescence, et d'imagerie par bioluminescence.

- Préférentiellement le marqueur est un nucléide radioactif. Des exemples de nucléides radioactifs plus particulièrement utilisés en imagerie nucléaire incluent le technétium 99m (^{99m}Tc), l'iode 123 (¹²³I), l'indium 111 (^{111m}In), le fluor 18 (¹⁸F), le gallium 67 (⁶⁷Ga), l'iode 125 (¹²⁵I), ou tout autre nucléide radioactif utilisable chez l'homme. Le marqueur peut être fixé au traceur par substitution (par exemple lorsque le ligand est une protéine, en substituant un H par un I vau niveau des résidus tyrosine), par complexion ou par chélation.

Avantageusement, le ligand de VCAM-1 est donc associé à un marqueur qui permet de localiser ou de faciliter la localisation du ligand de VCAM-1 dans un organisme auquel ledit agent de contraste a été administré. Le marqueur peut être sélectionné dans le groupe constitué d'un nucléide radioactif tel que ¹²³I, ¹²⁵I, ^{99m}Tc, ¹¹¹In, ^{1B}F, ⁶⁷Ga, d'un fluorophore tel que Fluorescine, Alexa, Cyanine, d'un composé chemiluminescent tel que le luminol, d'un composé bioluminescent tel que la luciférase, la phosphatase alkaline, ou d'un agent de contraste IRM tel que des nanoparticules ou du Gadolinium. Le choix du marqueur approprié, qui est fonction de la technique d'imagerie médicale utilisée in fine, est à la portée de l'homme du métier. Selon un mode de réalisation préférentiel, le ligand de VCAM-1 est marqué par un radionucléide.

Préférentiellement encore, ledit ligand de VCAM-1 marqué est sélectionné dans le groupe constitué de [¹²⁵I]-B 2702.84-75/75-84, [¹²⁵I]-B 2702.84-75, [¹²³I]-B 2702.84-75175-84, [¹²³I]-B 2702.84-75, [^{99m}Tc]-B 2702.84-75/75-84, [^{99m}Tc]-B 2702.84-75, [¹²³I]-4(B 2702.84-75)Raft, [¹²⁵I]-4(B 2702.84-75)Raft, et [^{99m}Tc]-4(B 2702.84-75)Raft, d'aptamères ligands de VCAM marqués à l'iode 123, à l'iode 125 ou au Tc99m. L'imagerie médicale au sens de l'invention peut être une technique d'imagerie nucléaire, d'IRM, d'imagerie optique. Préférentiellement l'imagerie médicale est une technique d'imagerie nucléaire telle que la scintigraphie.

La demande décrit également un agent de contraste comprenant un ligand de VCAM-1, éventuellement marqué, tel que décrit ci-dessus.

### Utilisation de ligands de VCAM-1

L'apparition de plaques d'athérome est une marque de l'athérosclérose, qui constitue en elle-même une maladie cardiovasculaire et peut générer différentes complications cardiovasculaires. La détection de plaques d'athérome constitue donc la caractérisation d'une maladie cardiovasculaire. D'autre part, la possibilité de suivre par imagerie l'évolution, c'est-à-dire la progression ou la régression, d'une plaque d'athérome précédemment identifiée représente un modalité pour évaluer l'efficacité d'un traitement thérapeutique chez un patient chez qui on a diagnostiqué une maladie cardiovasculaire.

Selon un mode de réalisation, la demande décrit donc l'utilisation d'un ligand de VCAM-1 pour la fabrication d'un agent de contraste utile pour la détection d'une maladie cardiovasculaire et/ou d'un risque d'accident ischémique aigu chez un sujet susceptible de présenter une telle maladie.

Préférentiellement, l'agent de contraste est utilisé pour détecter une athérosclérose, en particulier pour détecter une plaque d'athérome. Il peut s'agir d'une plaque d'athérome vulnérable.

Plus spécifiquement ladite plaque peut être une plaque d'athérome coronaire vulnérable. L'utilisation d'un ligand de VCAM-1 pour la fabrication d'un agent de contraste est donc utile pour la détection d'une maladie coronarienne, en particulier d'un angor stable ou instable. En outre, la présence d'une plaque d'athérome coronaire, surtout s'il s'agit d'une plaque vulnérable, expose le sujet à un risque d'accident ischémique aigu, notamment un infarctus du myocarde. L'utilisation d'un ligand de VCAM-1 pour la fabrication d'un agent de contraste est donc utile pour la détection d'un risque d'infarctus du myocarde chez un sujet.

La plaque d'athérome peut aussi être localisée au niveau d'une artère carotide (plaque d'athérome carotidienne). Ces lésions conduisent à des accidents vasculaires cérébraux, hémorragiques (rupture d'anévrisme) ou ischémiques (infarctus cérébral). L'utilisation d'un ligand de VCAM-1 pour la fabrication d'un agent de contraste est donc également utile pour la détection d'un risque d'accident vasculaire cérébral chez un sujet.

Il peut encore s'agir d'une plaque d'athérome localisée au niveau d'une artère rénale, le rein étant l'un des organes cibles de l'athérosclérose. Une sténose importante peut conduire à une hypertension artérielle et/ou à une insuffisance rénale. L'atteinte athéromateuse des artères-rénales peut conduire à un accident vasculaire aigu, l'embolie rénale. La demande décrit donc également l'utilisation d'un ligand de VCAM-1 pour la fabrication d'un agent de contraste utile pour la détection d'une hypertension artérielle et/ou à une insuffisance rénale, et/ou pour la détection d'un risque d'embolie rénale chez un sujet.

La présente invention concerne ainsi également l'utilisation d'un ligand de VCAM-1 comme agent de contraste dans l'imagerie médicale *in vivo,* non invasive, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEO ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé,
dans laquelle l'agent de contraste est utile pour la détection d'une maladie cardiovasculaire et/ou d'un risque d'accident ischémique aigu chez un sujet susceptible de présenter une telle maladie.

Les plaques athéromateuses peuvent être localisées au niveau des artères des membres inférieurs (risque d'ischémie aiguë d'un membre), on de l'aorte (risque de rupture d'anévrysme/dissection aortique). La demande décrit alors l'utilisation d'un ligand de VCAM-1 pour la fabrication d'un agent de contraste utile pour la détection d'un risque d'ischémie aiguë d'un membre ou d'un de rupture d'anévrysme aortique chez un sujet.

Selon un mode de réalisation, la demande décrit l'utilisation d'un ligand de VCAM-1 pour la fabrication d'un agent de contraste utile pour le suivi thérapeutique d'une maladie cardiovasculaire chez un sujet chez qui une maladie cardiovasculaire a été diagnostiquée.

Par suivi thérapeutique on entend l'observation de la réponse du sujet au traitement qui lui est administré. L'effet thérapeutique d'un traitement est généralement associé à un ralentissement ou une inhibition de la progression d'un maladie, à une réversion de la maladie, ou d'un ou plusieurs symptômes associés à cette maladie. Inversement, une absence d'effet thérapeutique peut se traduire par une stabilité, voire une accélération, de la progression de la maladie ou d'un ou plusieurs de ses symptômes.

Par exemple si la maladie cardiovasculaire au sens de l'invention est une plaque d'athérome, le suivi thérapeutique peut être réalisé en observant la disparition, la régression, le maintien, ou la croissance de la plaque d'athérome. Ainsi l'utilisation titre ou décrite ici peut comprendre les étapes consistant à :
a) administrer un ligand de VCAM-1 à un sujet chez qui on a détecté une plaque d'athérome ;
b) détecter la liaison du ligand de VCAM-1 au niveau de ladite plaque d'athérome ;
c) répéter les étapes a) et b) avant et après administration d'un traitement audit sujet ;
une absence ou une diminution de la liaison du ligand de VCAM-1 au niveau de ladite plaque étant indicatrice d'un traitement ayant un effet thérapeutique.

Dans le cadre de la présente demande, un "sujet" désigne un mammifère humain ou non humain, tel qu'un rongeur (rat, souris, lapin), un primate (chimpanzé), un félin (chat), un canin (chien). De préférence le sujet selon l'invention est un humain.

### Méthode de diagnostic

La demande décrit également une méthode de diagnostic d'une maladie cardiovasculaire et/ou de détection d'un risque d'un risque d'accident ischémique aigu chez un sujet susceptible de présenter une maladie cardiovasculaire, ladite méthode comprenant les étapes consistant à administrer un ligand de VCAM-1 audit sujet et à détecter ledit ligand de VCAM-1 dans l'organisme dudit sujet, la détection d'une localisation préférentielle dudit ligand de VCAM-1 au niveau du système cardiovasculaire étant indicatrice d'une maladie cardiovasculaire et/ou d'un risque d'accident ischémique aigu.

Par "localisation préférentielle" on entend que la quantité de ligand de VCAM-1 détecté au niveau du système cardiovasculaire est supérieur au bruit de fond qui correspond à une localisation non spécifique du ligand de VCAM-1 dans l'organisme.

Préférentiellement, le ligand de VCAM-1 est sélectionné dans le groupe constitué du peptide B 2702.84-75/75-84, du peptide B 2702.84-75, du composé 4(B 2702.84-75)Raft, et d'aptamères ligands de VCAM.

Avantageusement, le ligand de VCAM-1 est associé à un marqueur qui permet de localiser ou de faciliter la localisation du ligand de VCAM-1 dans un organisme auquel ledit agent de contraste a été administré.

Le marqueur peut être sélectionné dans le groupe constitué d'un nucléide radioactif tel que le technétium 99m (^{99m}Tc), l'iode 123 (¹²³I), l'indium 111 (^{111m}In), le fluor 18 (¹³F), le gallium 67 (⁶⁷Ga) l'iode 125 (¹²⁵I), ou tout autre nucléide radioactif utilisable chez l'homme, d'un fluorophore tel que Fluorescine, Alexa, Cyanine, d'un composé chemiluminescent tel que Fluorescine, Alexa, Cyanine, d'un composé chemiluminescent tel que le luminol, d'un composé bioluminescent tel que la luciférase ou la phosphatase alkaline et d'un agent de contraste IRM tel que des nanoparticules ou du Gadolinium. Selon un mode de réalisation préférentiel, le ligand de VCAM-1 est marqué par un nucléide radioactif.

Préférentiellement, le ligand de VCAM-1 marqué est sélectionné dans le groupe constitué de [¹²⁵I]-B 2702.84-75175-84, [¹²⁵]-B 2702.84-75, [¹²⁵I]-B 2702.84-75/75-84, [¹²³I]-B 2702.84-75, [^{99m}Tc]-B 2702.84-75/75-84, [^{99m}Tc]-B 2702.84-75, [¹²³I]-4(B 2702.84-75)Raft, [¹²⁵I]-4(B 2702.84-75)Raft, et [^{99m}Tc]-4(B 2702.84-75)Raft, d'aptamères ligands de VCAM marqués à l'Iode 123, à l'Iode 125 ou au Tc99m.

Le ligand de VCAM-1 peut être détecté par tout moyen connu de l'homme du métier. En particulier, le choix d'une technique d'imagerie appropriée, en fonction du type de marqueur éventuellement associé au ligand de VCAM-1, est à la portée de l'homme du métier. Avantageusement, la localisation du ligand de VCAM-1 est effectuée au moyen d'une technique non invasive telle que l'imagerie nucléaire, en particulier la scintigraphie, ou l'IRM.

Le ligand de VCAM-1 peut être administré par exemple par voie orale, par inhalation, par voie parentérale (en particulier par injection intraveineuse), sous une forme appropriée. Lorsque la voie parentérale est envisagée, le ligand de VCAM-1 peut être sous la forme de solutés et suspensions injectables conditionnées en ampoules ou flacons. Les formes pour l'administration parentérale sont obtenues de façon conventionnelle par mélange du ligand de VCAM-1 avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses. A titre de tampon, l'homme du métier pourra utiliser des tampons à base de sels de phosphate organique. Des exemples d'agents de mise en suspension englobent la méthylcellulose, l'hydroxyéthylcellulose, l'hydroxy-propylcellulose, l'acacia et. la carboxyméthylcellulose sodique. En outre, des stabilisants utiles selon l'invention sont le sulfite de sodium et le métasulfite de sodium, tandis que l'on peut citer le p-hydroxybenzoate de sodium, l'acide sorbique, le crésol et le chlorocrésol en tant que conservateurs.

La quantité de ligand de VCAM-1 administrée dépend naturellement du mode d'administration, de la taille et/ou du poids du patient, et de la technique de détection utilisée.

Par exemple la dose de ligand utilisée pour l'imagerie nucléaire est préférentiellement comprise entre environ 20 et environ 500 microgrammes.

### Méthode de traitement

La demande décrit en outre l'utilisation d'un ligand de VCAM-1 pour la fabrication d'un médicament destiné au traitement d'une maladie cardiovasculaire. Une méthode de traitement d'une maladie cardiovasculaire comprenant l'administration d'une quantité thérapeutiquement efficace d'un ligand de VCAM-1 est également décrite ici :

La présente invention concerne également l'utilisation d'un ligand de VCAM-1 pour la fabrication d'un médicament destiné au traitement thérapeutique ou préventif d'une maladie cardiovasculaire, dans laquelle ledit ligand de VCAM-1 est utilisé pour traiter une plaque d'athérome coronaire ou aortique vulnérable, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé; ledit ligand de VCAM-1 étant couplé à un agent cytotoxique.

Elle concerne aussi un ligand de VCAM-1 pour son utilisation pour le traitement thérapeutique ou préventif d'une maladie cardiovasculaire, ledit ligand de VCAM-1 étant utilisé pour traiter une plaque d'athérome coronaire ou aortique vulnérable, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé;
ledit ligand de VCAM-1 est couplé à un agent cytotoxique.

L'utilisation d'un ligand de VCAM-1, couplé à un agent cytotoxique, permet en effet de détruire sélectivement les cellules inflammatoires qui expriment VCAM-1 et qui contribuent à l'instabilité des plaques d'athérosclérose.

Par "traitement" d'une maladie cardiovasculaire on entend le "traitement thérapeutique" (ou curatif) d'une maladie cardiovasculaire, qui inclut le ralentissement ou l'inhibition de l'évolution d'une plaque d'athérosclérose, en particulier vers un stade de plaque d'athérosclérose vulnérable, ou la régression d'une plaque d'athérosclérose, en particulier d'une plaque vulnérable ; on entend également le "traitement prophylactique" d'une maladie cardiovasculaire qui inclut notamment la prévention d'un accident ischémique aigu.

Préférentiellement, le ligand de VCAM-1 est sélectionné dans le groupe constitué du peptide B 2702.84-75/75-84, du peptide B 2702.84 -75,du composé 4(B 2702.84-75)Raft, et d'aptamères ligands de VCAM.

Le ligand de VCAM-1 est avantageusement couplé à un agent cytotoxique, de manière à mettre en contact l'agent cytotoxique avec une cellule exprimant VCAM-1 et donc de détruire sélectivement celle-ci. Le choix d'un agent cytotoxique approprié est à la portée de l'homme du métier. Des exemples d'agents cytotoxiques incluent notamment des isotopes radioactifs, des toxines ou des agents chimiothérapeutiques tels que la doxorubicine ou le taxotère, par exemple.

Selon un mode de réalisation le ligand de VCAM-1 est couplé à un isotope radioactif de manière à réaliser une radiothérapie, curative et/ou préventive, d'une maladie cardiovasculaire. La radiothérapie doit mettre en oeuvre des rayonnements dont le parcours moyen est faible afin de déposer l'essentiel de leur énergie au contact du tissu ou des cellules cibles. C'est pourquoi l'isotope radioactif est de préférence choisi parmi les émetteurs β⁻, tels que l'Iode 131 (¹³¹I) et l'Yttrium 90 (⁹⁰Yt).

La méthode de traitement selon l'invention peut avantageusement être mise en oeuvre en utilisant un ligand de VCAM-1 couplé à un isotope radioactif sélectionné dans le groupe constitué de [¹³¹]-B 2702.84-75/75-84, [¹³¹I]-B 2702.84-75, [⁹⁰Yt]-B 2702.84-75/75-84, [⁹⁰Yt]-B 2702.84-75, [¹³¹I]-4(B 2702.84-75)Raft, [⁹⁰Yt]-4(B 2702.84-75)Raft, et d'aptamères ligands de VCAM marqués à l'Iode 131 ou à l'Yttrium 90.

Selon un autre mode de réalisation, le ligand de VCAM-1 peut être couplé à une toxine, par exemple la ricine ou une toxine bactérienne, telle que la saporine ou la toxine du choléra.

La demande décrit plus particulièrement l'utilisation d'un ligand de VCAM-1 pour la fabrication d'un médicament destiné au traitement de l'athérosclérose et/ou à la prévention d'un accident ischémique aigu chez un sujet susceptible de présenter une maladie cardiovasculaire.

Préférentiellement, le ligand de VCAM-1 est utilisé pour traiter une plaque d'athérome vulnérable, de préférence encore une plaque d'athérome coronaire ou aortique vulnérable.

Préférentiellement, ledit accident ischémique aigu est sélectionné dans le groupe constitué d'un infarctus du myocarde, un accident vasculaire cérébral, une embolie rénale, une ischémie aiguë d'un membre, et une rupture d'anévrysme aortique.

Le ligand de VCAM-1 peut être administré par exemple par voie orale, par inhalation, par voie parentérale (en particulier par injection intraveineuse), sous une forme appropriée. Lorsque la voie parentérale est envisagée, le ligand de VCAM-1 peut être sous la forme de solutés et suspensions injectables conditionnées en ampoules ou flacons. Les formes pour l'administration parentérale sont obtenues de façon conventionnelle par mélange du ligand de VCAM-1 avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses. A titre de tampon, l'homme du métier pourra utiliser des tampons à base de sels de phosphate organique. Des exemples d'agents de mise en suspension englobent la méthylcellulose, l'hydroxyéthylcellulose, l'hydroxy-propylcellulose, l'acacia et la carboxyméthylcellulose sodique. En outre, des stabilisants utiles selon l'invention sont le sulfite de sodium et le métasulfite de sodium, tandis que l'on peut citer le p-hydroxybenzoate de sodium, l'acide sorbique, le crésol et le chlorocrésol en tant que conservateurs.

La quantité de ligand de VCAM-1 administrée dépend naturellement du mode d'administration, de la taille et/ou du poids du patient, et de la nature de l'agent cytotoxique qui peut lui être associé. Par exemple lorsque le ligand de VCAM-1 est marqué, par exemple à l'iode 131, on peut administrer environ 25 mg de ligand de VCAM-1 par m² de surface corporelle, ce qui correspond à une activité injectée de 50 mCi d'lode 131.

Les exemples et figures suivantes illustrent l'invention sans en limiter sa portée.

### LEGENDE DES FIGURES

La figure 1 montre les résultats d'analyse de la biodistribution tissulaire des traceurs [¹²⁵I]-B2702.84-75/75-84 et [¹²⁵I]-B2702.84-75 chez le lapin hyperlipidémique WHHL.
La figure 2 montre la structure du composé 4(B 2702.84-75)Raft.
La figure 3 montre l'activité aortique en ¹²³I-82702.84-75-(123I-B2702-p) et ^{99m}Tc-B2702.84-75 (^{99m}Tc-B2702-p) normalisée à l'activité sanguine, 180 minutes après l'injection des traceurs chez des animaux contrôles et WHHL. * P < 0.05 vs. Contrôles.
La figure 4 montre la cinétique sanguine de ¹²³I-B2702.84-75 et ^{99m}Tc-B2702.84-75.
La figure 5 montre la quantification des activités aortiques en ¹²³I-B2702.84-75 (A) et ^{99m}Tc-B270284-75 (B) à partir des images autoradiographiques *ex vivo* réalisées 180 minutes après l'injection des traceurs chez les animaux contrôles et dans les zones saines (Sudan IV-négatives) ou les zones d'accumulation lipidiques (Sudan IV-positives) des animaux WHHL. * P < 0.05 vs. Contrôles, t P < 0.5 vs. Zones Sudan IV-négatives.

### EXEMPLE 1 : Matériel et méthodes

La spécificité des ligands de VCAM-1 vis-à-vis de plaques d'athérome vulnérables a été étudiée sur un modèle animal.

### Modèle animal

Le modèle biologique utilisé pour l'évaluation de la capacité de ligands de VCAM-1 à se lier aux plaques d'athérome est le lapin génétiquement hyperlipidémique de souche WHHL (Watanabe Heritable Hyperlipidemic), qui représente un modèle de choix pour le développement de l'athérosclérose (Clubb et al. (2001)). Les animaux ont été obtenus auprès du Centre de Production Animale (Olivet, France).

### Synthèse des peptides

Le ligand de VCAM-1 choisi pour cette étude est la molécule HLA-B2702. Ling et al. (2000) ont montré que le motif constitué par les résidus 75 à 84 de HLA-B2702 (B2702.84-75) ainsi que ce même motif répété (B2703.84-75/75-84) se liaient spécifiquement à VCAM-1.

Les peptides 82702.84-75 [YRLAIRLNER], B2703.84-75/75-84 [YRLAIRLNERRENLRIALRY] et un peptide B2702.84-75 modifié pour le marquage au technétium, B2702-His [YRLAIRLNERGH] ont été préparés par synthèse en phase solide. L'assemblage des peptides protégés a été effectué soit manuellement, en utilisant une stratégie Fmoc/tBu dans un réacteur en verre muni d'un fritté de verre, soit automatiquement sur un synthétiseur (348 Ω synthesizer, Advance ChemTech). Les réactions de couplage ont été effectuées en utilisant 1.5-2 eq. d'acides aminés protégés par le N-α-Fmoc, activés in situ avec 1.5-2 eq. De PyBOP et 3-4 eq. de DIEA dans du DMF (10 ml/g de résine) pendant 30 min (les proportions sont données par rapport à la charge en résine). Avec un couplage manuel, les synthèses sont contrôlées par des tests de Kaiser et/ou au TNBS. Les groupes protecteurs N-α-Fmoc ont été enlevés par traitement avec une solution de pipéridine:DMF (1:4) (10 ml/g de résine) pendant 10 min. Le traitement est répété trois fois et la déprotection complète a été contrôlée par mesure de l'absorption UV à 299 nm des lavages à la pipéridine. Les peptides synthétiques linéaires ont été récoltés directement après clivage acide (1 % TFA dans CH₂Cl₂). Les résines ont été traitées pendant 3 min de façon répétée jusqu'à ce que les billes de résines virent au violet foncé. Les lavages ont été rassemblés et concentres sous pression réduite et des peptides solides blancs ont été obtenus par précipitation à l'éther puis analysé par RP-HPLC.

### Radiomarquage

Le B2703.84-75/75-84 a été marqué à l'¹²⁵I (Amersham radiochemical center) et B2702.84-75 avec ¹²⁵I or ¹²³I (Schering SA) sur la Tyrosine (acide aminé en position 84) en utilisant de la chloramine-T. Brièvement, 74 MBq de ¹²⁵I ou 200 MBq de ¹²³I ont été ajouté à 20 ou 40 nmoles de peptide et 20 ou 80 µl de chloramine T préparée extemporanément (1mg/ml), respectivement. La réaction a été stoppée après 15 minutes par addition de 20 µl ou 80µl de métabisulfite de sodium (4 mg/ml) pour ¹²⁵I et ¹²³I, respectivement. Une analyse par chromatographie en couche mince (CCM) (RP-18R254; Merck) avec un mélange acétonitrile/H₂O (60/40) comme éluant permet d'évaluer la pureté radiochimique.

Deux acides aminés [HG] ont été ajoutés au peptide B2702.84-75 pour le marquage par le ^{99m}Tc sur l'histidine avec [^{99m}Tc(OH₂)₍₃₎ (CO)₍₃₎] en utilisant un système de ligand tridenté. Le précurseur [^{99m}Tc(OH₂)₍₃₎ (CO)₍₃₎] a été synthétisé en utilisant un "tricarbonyl pharmaceuticals Kit" (Isolink^{®}, Mallinckrodt). Le kit a été reconstitué avec 2 GBq de ^{99m}TcO₄⁻ (Schering SA), et incubé à 100°C pendant 20 minutes. Après ajustement du pH à 8,0, 800 MBq de cette solution ont été ajoutés à 30 nmoles de B2702.84-75 modifié et incubés pendant 20 minutes à 80°C.

### Protocole expérimental

Pour les expériences décrites ci-dessous, B2702.84-75/75-84 et B2702.84-75, marqués à l'iode-125 ([¹²⁵I]-B2702.84-75/75-84 et [¹²⁵I]-82702.84-75) par substitution électrophile, sont injectés de manière intraveineuse (∼ 120 µCi/kg) à des animaux normaux ou hyperlipidémiques (n=4/groupe) de poids et d'âge comparables préalablement anesthésiés par injection intramusculaire d'un mélange xylazine (5 mg/kg)/kétamine (35 mg/kg) et cathétérisés dans les veines controlatérales de l'oreille.

Des prélèvements sanguins ont été réalisés à 1, 2, 5, 10, 15, 20, 25 et 30 minutes après injection des traceurs. Les animaux ont été euthanasiés 30 minutes (exemples 2, 3 et 4) ou 180 minutes (exemples 5 et 6) après injection des ligands de VCAM-1 marqués. Des échantillons des principaux organes ont été prélevés. Ces échantillons, ainsi que les prélèvements sanguins, ont été comptés pour la radioactivité qu'ils contiennent. L'aorte dorsale, la crosse aortique et l'aorte ventrale ont également été prélevées et immédiatement placées dans la formaline à 10 %. Une portion d'aorte ventrale a été placée 24 heures dans du formaldéhyde 3,7 % pour une inclusion ultérieure en paraffine et la vérification par immunohistologie de la présence de VCAM-1.

Les aortes dorsales et ventrales ont été disposées au contact d'un écran phosphore haute résolution (Fujifilm 08SR2025) à l'obscurité pendant 18 heures. L'écran a ensuite été scanné (phosphoimager Fujifilm BAS 5000) et les images de la répartition des ligands de VCAM-1 marqués sur les aortes d'animaux sains ou hyperlipidémiques ont été quantifiées grâce au logiciel Scion Image Beta disponible sur le site Internet du National Institute of Health.

Finalement, une coloration au Sudan IV a été réalisée sur les aortes dorsales et ventrales et a permis de comparer les régions de fixation préférentielle des traceurs avec les zones de forte concentration en lipides correspondant aux régions de développement des plaques d'athérome.

### EXEMPLE 2 : Validation du modèle expérimental

La coloration rouge au Sudan IV a permis de mettre en évidence la présence de plages lipidiques correspondant aux plaques d'athérome sur les aortes des animaux hyperlipidémiques. Ces plages lipidiques étaient absentes chez les animaux normolipidémiques. L'étude immunohistologique a confirmé l'expression de VCAM-1 sur les plaques athéromateuses des lapins WHHL et son absence dans les vaisseaux des animaux normolipidémiques. Le modèle utilisé était donc adapté à l'étude de la fixation *in vivo* des ligands de VCAM-1 [¹²⁵I]-B2702.84-75175-84 et [¹²⁵I]-B2702.84-75.

### EXEMPLE 3 : Biodistribution et activité sanguine (euthanasie après 30 minutes)

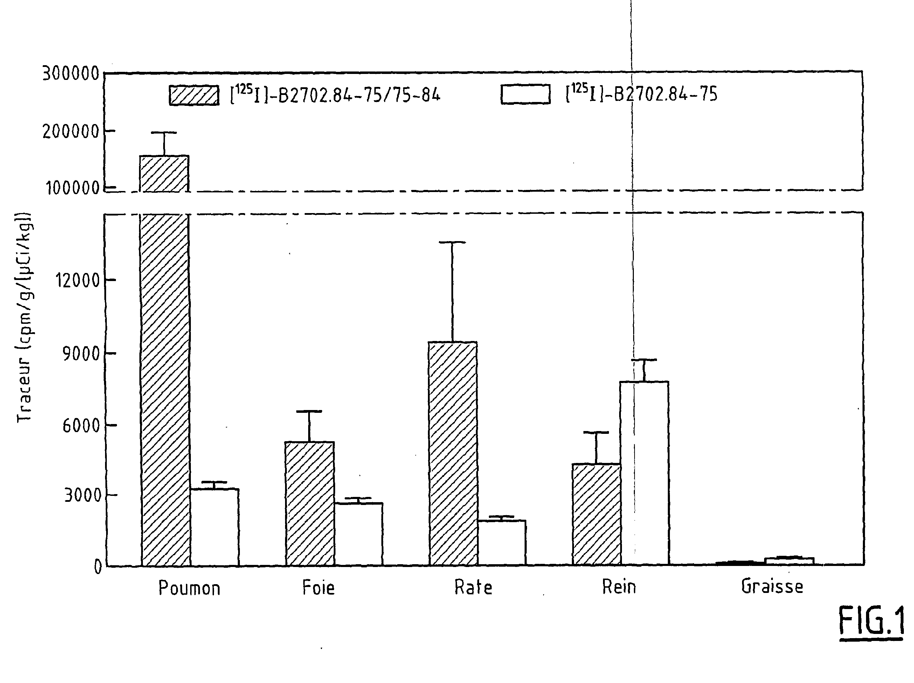

Les résultats de l'étude de biodistribution ont indiqué que [¹²⁵I]-B2702.84-75/75-84 se fixe de manière similaire dans le poumon, le foie, la rate, le rein et la graisse chez les animaux normaux et hyperlipidémiques, avec une très forte fixation pulmonaire. La fixation pulmonaire de [¹²⁵I]-B2702.84-75 est environ 50 fois moindre, avec une activité globalement similaire à celle de [¹²⁵I]-B2702.84-75/75-84 dans les autres organes des lapins WHHL (figure 1).

Les activités sanguines des deux traceurs ont atteint un plateau 10 minutes après injection, qui représentait 40 % de l'activité initiale. L'activité circulante observée pour [¹²⁵I]-B2702.84-75 était alors deux fois supérieure à celle de [¹²⁵I]-B2702.84-75/75-84.

### EXEMPLE 4 : Fixation aortique (euthanasie après 30 minutes)

L'analyse qualitative des images autoradiographiques indique que les deux traceurs sont préférentiellement fixés sur les plages lipidiques révélées par la coloration au Sudan IV et qui correspondaient aux plaques d'athérome chez les lapins WHHL. Le traceur [¹²⁵I]-B2702.84-75/75-84 n'a montré aucune fixation sur les aortes des animaux sains.

La quantification des images autoradiograhiques a révélé que le rapport de fixation de [¹²⁵I]-B2702.84-75/75-84 entre les plages lipidiques et les zones aortiques normales des lapins WHHL était de 7,9±2,0, tandis que le rapport de l'activité en traceur entre les régions de plus forte et de plus faible fixation sur les aortes des lapins normaux était de 1,2±0,1 (p<0,01 vs groupe WHHL). Le rapport de fixation plaque d'athérome/zone aortique normale de [¹²⁵I]-B2702.84-75 chez les animaux WHHL était de 3,0±0,3.

En conclusion, les résultats indiquent que tes deux-traceurs se fixent préférentiellement sur les plaques d'athérome exprimant VCAM-1. Les rapports de fixation du traceur du niveau de la plaque d'athérome sur la fixation au niveau de la zone aortique normale sont plus favorables à [¹²⁵I]-B2702.84-75/75-84. Toutefois, le traceur [¹²⁵I]-B2702.84-75 ne présentait pas de fixation pulmonaire notable, ce qui en fait un composé particulièrement avantageux pour l'imagerie de la plaque d'athérome coronaire vulnérable.

### EXEMPLE 5 : Biodistribution et activité sanguine (euthanasie après 180 minutes)

Les traceurs ¹²³I-B2702.84-75 et ^{99m}Tc-B2702.84-75 ont été évalués selon la même méthodologie que celle utilisée pour les exemples 3 et 4 à la différence que les animaux étaient euthanasiés 180 minutes (30 minutes auparavant) après l'injection des traceurs.

Les traceurs se fixent de manière similaire dans les organes des lapins contrôles et WHHL, excepté dans l'aorte où une tendance à l'augmentation est observée chez les animaux WHHL (tableau 1).

**Tableau 1 : Biodistribution de ¹²³I-B2702.84-75 et ^{99m}Tc-B2702.84-75 180 minutes après l'injection :**

| | ¹²³I-B2702.84-75 | | ^{99m}Tc-B2702.84-75 | |
|---|---|---|---|---|
| Tissu | Contrôle | WHHL | Contrôle | WHHL |
| | (n = 3) | (n = 3) | (n = 3) | (n = 3) |
| Sang | 0,190± 0,038 | 0,161±0,048 | 0,097±0,017 | 0,102±0,033 |
| Coeur | 0,088±0,017 | 0,088±0,031 | 0.029±0,008 | 0, 044±0,015 |
| Aorte | 0,011±0,004 | 0,034±0,012 | 0,025±0,009 | 0,046±0,012 |
| Poumon | 0,138±0,028 | 0,163±0,044 | 0,166±0,052 | 0.208±0,044 |
| Foie | 0,086±0,025 | 0,096±0,029 | 0,427±0,080 | 0,332±0.042 |
| Rate | 0.076±0,015 | 0,089±0,029 | 0,198±0,061 | 0,172±0,070 |
| Rein | 0,307±0,052 | 0,490±0,184 | 0,558±0,109 | 1,361±0,721 |
| Graisse | 0,011±0,001 | 0,015±0,006 | 0,012±0,001 | 0,019±0,009 |
| Muscle | 0,028±0,003 | 0,026±0,009 | 0,008±0,002 | 0.017±0,003 |
| Estomac | 0,095±0,025 | 0,114±0,040 | 0,045±0,009 | 0,052±0,015 |

Les valeurs de fixation sont exprimées en pourcentage de la dose injectée par gramme d'organe (% ID/g).

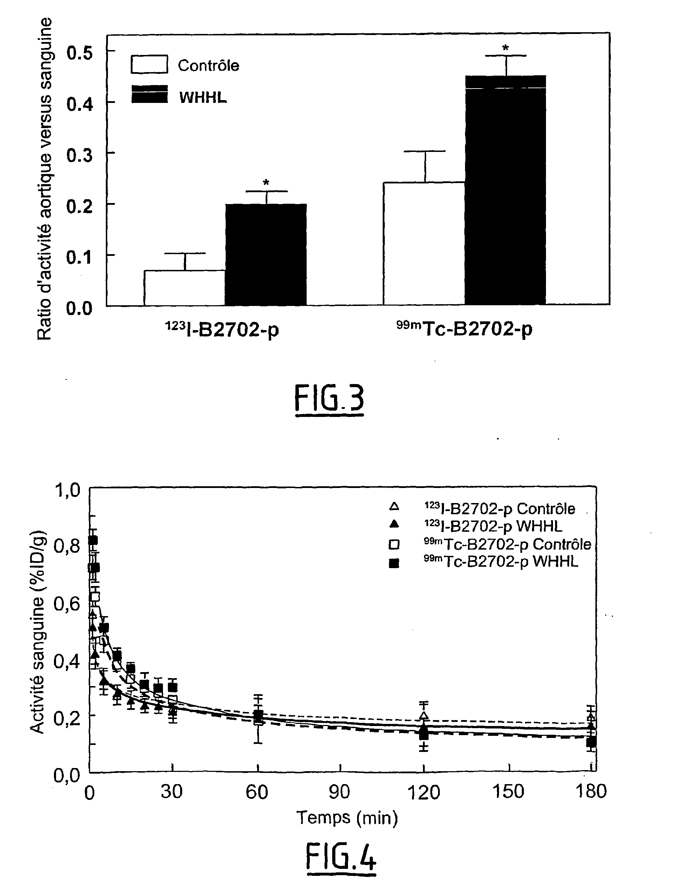

Cette augmentation devient significative pour les deux traceurs lorsque l'activité aortique est normalisée à l'activité circulante en traceur (figure 3).

Les prélèvements sanguins effectués pour mesurer l'activité circulante en traceur étaient réalisés à 1, 2, 5, 10, 20, 25, 30, 60, 120 et 180 minutes après l'injection. Les cinétiques sanguines sont présentées dans la figure 4. 180 minutes après l'injection, l'activité circulante en ¹²³I-B2702.84-75 et ^{99m}Tc-82702.84-75 représente respectivement 30 % et 15 % de l'activité initiale. Pour rappel, l'activité circulante en ¹²⁵I-B2702.84-75 représentait environ 40 % de l'activité initiale 30 minutes après l'injection dans les expériences décrites dans l'exemple 3.

### EXEMPLE 6 : fixation aortique (euthanasie après 180 minutes) :

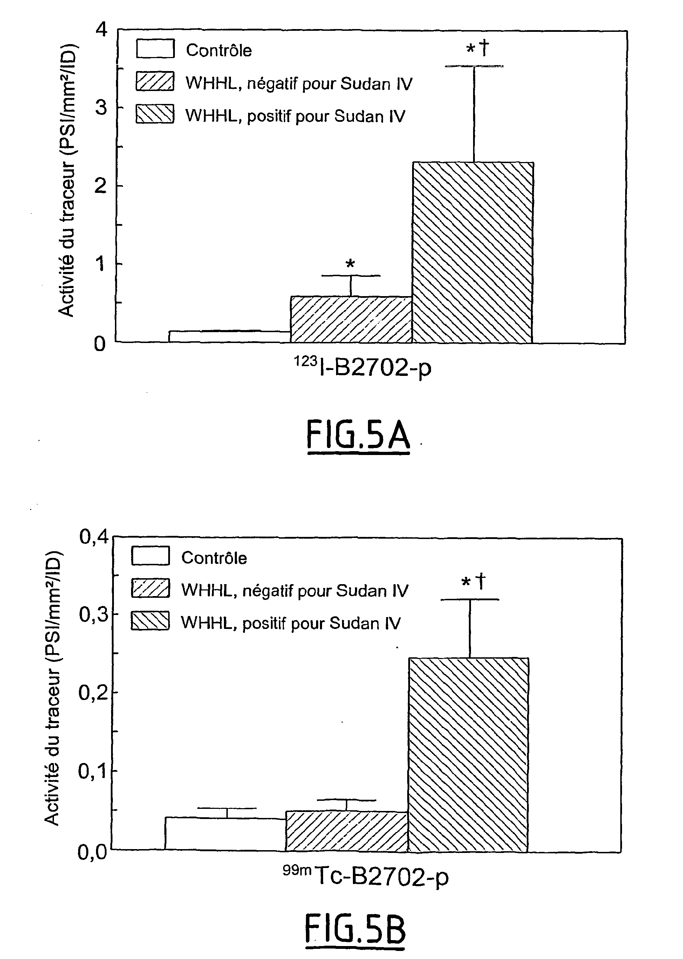

La quantification des images autoradiographiques indique que les deux traceurs sont préférentiellement fixés aux plages lipidiques révélées par la coloration au Sudan IV et correspondant aux plaques d'athérome. Les rapports de fixation plaque d'athérome/zone aortique normale de ¹²³I-82702.84-75 et ^{99m}Tc-82702.84-75 chez les animaux WHHL sont respectivement de 4.0 et 4.8. Ce rapport était de 3.0 dans les expériences précédentes réalisée 30 minutes après l'injection des traceurs. Enfin, les fixations de ¹²³I-B2702.84-75 et ^{99m}Tc-B2702.84-75 sur les plaques d'athérome des animaux WHHL sont 17.0 et 5.9 fois supérieures à celles observées sur les aortes des animaux contrôles. Ces résultats sont résumés dans les figures 5a et 5B ci-dessous.

### BIBLIOGRAPHIE

Clubb et al. (2001) Development of atherosclerotic plaque with endothelial disruption in Watanabe Heritable Hyperlipidemic rabbit aortas. Cardiovasc Pathol. 9:1-11
Colas P, Cohen B, Jessen T, Grishina 1, McCoy J, Brent R. (1996) Genetic selection of peptide aptamers that recognize and inhibit cyclin-dependent kinase 2. Nature, 380, 548-50.
Checovich W. J., Bolger R. E., Burke T.. (1995) Fluorescence polarization-a new tool for cell and molecular biology. Nature; 375(6528):254-6.
Fayad ZA et Fuster V (2001) Clinical imaging of the high-risk or vulnerable atherosclerotic plaque. Circ Res.; 89:305-316
Jayasena S.D. (1999) Aptamers: an emerging class of molecules that rival antibodies in diagnostics. Clin Chem. 45(9):1628-50.
Ling X et al. (2000) An immunosuppressive and anti-inflammatory HLA class I-derived peptide binds vascular cell adhesion molécule-1. Transplantation. 70 :662-667
Narula J et al. (1999) Strategic targeting of atherosclerotic lesions. J Nucl Cardiol. 6:81-90
Osborn L et al. (1989) Direct expression cloning of vascular cell adhesion molecule 1, a cytokine-induced endothelial protein that binds to lymphocytes. Cell. (6):1203-11
Tuerk C. and Gold L. (1990) Systematic evolution of ligands by exponential enrichment: RNA ligands to bacteriophage T4 DNA polymerase. Science. 3;249(4968):505-10.

## Revendications

1. Utilisation d'un ligand de VCAM-1 comme agent de contraste dans l'imagerie médicale *in vivo,* non invasive, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé.

2. Utilisation d'un ligand de VCAM-1 selon la revendication 1, dans laquelle l'imagerie médicale est une technique sélectionné dans le groupe constitué de la scintigraphie, de l'IRM, et de l'imagerie optique.

3. Utilisation d'un ligand de VCAM-1 selon la revendication 1 ou 2, dans laquelle le ligand de VCAM-1 est sélectionné dans le groupe constitué de B 2702-84.75175-84, B 2702.84-75, 4(B 2702.84-75)Raft, et d'aptamères ligands de VCAM-1.

4. Utilisation d'un ligand de VCAM-1 selon l'une quelconque des revendications 1 à 3, dans laquelle le ligand de VCAM-1 est associé à un marqueur.

5. Utilisation d'un ligand de VCAM-1 selon la revendication 4, dans laquelle ledit marqueur est sélectionné dans le groupe constitué d'un nucléide radioactif, d'un fluorophore, d'un composé chemiluminescent, d'un composé bioluminescent et d'un agent de contraste IRM.

6. Utilisation d'un ligand de VCAM-1 selon la revendication 4 ou 5, dans laquelle ledit marqueur est sélectionné dans le groupe constitué de ¹²³I, ¹²⁵I, 99^{m}Tc, ¹¹¹In, ¹⁸F, ⁶⁷Ga, de nanoparticules, du Gadolinium, de fluorescine, d'Alexa, de cyanine, de luciférase, et de phosphatase alkaline.

7. Utilisation d'un ligand de VCAM-1 selon l'une quelconque des revendications 1 à 6, dans laquelle le ligand de VCAM-1 est marqué et sélectionné dans le groupe constitué de [¹²⁵I]-B 2702.84-75/75-84, [¹²⁵I]-B 2702.84-75, [¹²³I]-B 2702.84-75/75-84, [¹²³I]-B 2702.84-75, [^{99m}Tc]-B 2702.84-75/75-84, [^{99m}Tc]-B 2702.84-75, [¹²³I]-4(B 2702.84-75)Raft, [¹²⁵I]-4(B 2702.84-75)Raft, [^{99m}Tc]-4(B 2702.84-75)Raft, et d'aptamères ligands de VCAM marqués à l'Iode 123, à l'Iode 125 ou au Tc99m.

8. Utilisation d'un ligand de VCAM-1 selon l'une quelconque des revendications 1 à 7, dans laquelle l'agent de contraste est utile pour la détection d'une maladie cardiovasculaire et/ou d'un risque d'accident ischémique aigu chez un sujet susceptible de présenter une telle maladie.

9. Utilisation d'un ligand de VCAM-1 selon la revendication 8, dans laquelle on détecte une plaque d'athérome.

10. Utilisation d'un ligand de VCAM-1 selon la revendication 9, dans laquelle la plaque d'athérome est une plaque d'athérome vulnérable.

11. Utilisation d'un ligand de VCAM-1 selon la revendication 9 ou 10 dans laquelle la plaque d'athérome est une plaque d'athérome coronarienne vulnérable.

12. Utilisation d'un ligand de VCAM-1 selon l'une quelconque des revendications 8 à 11, dans laquelle ladite maladie cardiovasculaire est une maladie coronarienne.

13. Utilisation d'un ligand de VCAM-1 selon l'une quelconque des revendications 8 à 12, dans laquelle ledit risque d'accident ischémique aigu est un risque d'infarctus du myocarde.

14. Utilisation d'un ligand de VCAM-1 selon l'une quelconque des revendications 8 à 10, dans laquelle la plaque d'athérome est une plaque d'athérome carotidienne, rénale ou localisée au niveau d'une artère des membres inférieurs.

15. Utilisation d'un ligand de VCAM-1 selon l'une quelconque des revendications 8 à 10 ou 14, dans laquelle ledit risque d'accident ischémique aigu est un risque d'embolie rénale.

16. Utilisation d'un ligand de VCAM-1 selon l'une quelconque des revendications 8 à 10 ou 14, dans laquelle ledit risque d'accident ischémique aigu est un risque d'accident vasculaire cérébral.

17. Utilisation d'un ligand de VCAM-1 selon l'une quelconque des revendications 1 à 7, dans laquelle l'agent de contraste est utile pour le suivi thérapeutique d'une maladie cardiovasculaire chez un sujet chez qui une maladie cardiovasculaire a été diagnostiquée.

18. Utilisation d'un ligand de VCAM-1 pour la fabrication d'un médicament destiné au traitement thérapeutique ou préventif d'une maladie cardiovasculaire, dans laquelle ledit ligand de VCAM-1 est utilisé pour traiter une plaque d'athérome coronaire ou aortique vulnérable, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé;
ledit ligand de VCAM-1 étant couplé à un agent cytotoxique.

19. Utilisation selon la revendication 18, dans laquelle ledit agent cytotoxique est un isotope radioactif sélectionné dans le groupe constitué de l'Iode 131 et de l'Yttrium 90 (⁹⁰Yt).

20. Utilisation selon l'une quelconque des revendications 18 à 19, dans laquelle ledit ligand de VCAM-1 est sélectionné dans le groupe constitué du peptide B 2702.84-75/75-84, du peptide B 2702.84-75, du composé 4(B 2702.84-75)Raft, et d'aptamères ligands de VCAM.

21. Utilisation selon l'une quelconque des revendications 18 à 20, dans laquelle ledit ligand de VCAM-1 est utilisé pour la prévention d'un accident ischémique aigu sélectionné dans le groupe constitué d'un infarctus du myocarde, un accident vasculaire cérébral, une embolie rénale, une ischémie aiguë d'un membre, et une rupture d'anévrysme aortique.

22. Ligand de VCAM-1 pour son utilisation pour le traitement thérapeutique ou préventif d'une maladie cardiovasculaire, ledit ligand de VCAM-1 étant utilisé pour traiter une plaque d'athérome coronaire ou aortique vulnérable, le ligand de VCAM-1 étant choisi dans le groupe constitué du peptide constitué de la séquence YRLAIRLNER (SEQ ID NO: 1) et de ses dérivés, du peptide constitué de la séquence RENLRIALRY (SEQ ID NO: 5) et de ses dérivés, du peptide constitué de la séquence YRLAIRLNERRENLRIALRY (SEQ ID NO: 2) et de ses dérivés, du composé 4(B2702.84-75)Raft et de ses dérivés et des aptamères ligands de VCAM-1; les dérivés des peptides et du composé étant un peptide ou un composé dans lequel la séquence RENLRIALRY (SEQ ID NO: 5) a été modifiée par ajout, suppression, substitution ou modification d'un acide aminé;
ledit ligand de VCAM-1 est couplé à un agent cytotoxique.

23. Ligand de VCAM-1 selon la revendication 22, dans lequel ledit agent cytotoxique est un isotope radioactif sélectionné dans le groupe constitué de l'Iode 131 et de l'Yttrium 90 (⁹⁰Yt).

24. Ligand de VCAM-1 selon l'une quelconque des revendications 22 à 23, ledit ligand de VCAM-1 étant sélectionné dans le groupe constitué du peptide B 2702.84-75/75-84, du peptide B 2702.84-75, du composé 4(B 2702.84-75)Raft, et d'aptamères ligands de VCAM.

25. Ligand de VCAM-1 selon l'une quelconque des revendications 22 à 24, ledit ligand de VCAM-1 étant utilisé pour la prévention d'un accident ischémique aigu sélectionné dans le groupe constitué d'un infarctus du myocarde, un accident vasculaire cérébral, une embolie rénale, une ischémie aiguë d'un membre, et une rupture d'anévrysme aortique.

## Claims

1. Use of a VCAM-1 ligand as a contrast agent for non-invasive medical imaging *in vivo,* the VCAM-1 ligand being selected from the group consisting of the peptide consisting of the sequence YRLAIRLNER (SEQ ID NO: 1) and its derivatives, the peptide consisting of the sequence RENLRIALRY (SEQ ID NO: 5) and its derivatives, the peptide consisting of the sequence YRLAIRLNERRENLRIALRY (SEQ ID NO: 2) and its derivatives, the composition 4 (B2702.84-75)Raft and its derivatives and VCAM-1 ligand aptamers, the derivatives of a peptide and a compound being a peptide or a compound wherein the sequence RENLRIALRY (SEQ ID NO: 5) has been modified by addition, deletion, substitution, or modification of an amino acid.

2. Use of a VCAM-1 ligand according to Claim 1, in which the medical imaging is a technique selected from the group consisting of scintigraphy, MRI and optical imaging.

3. Use of a VCAM-1 ligand according to Claim 1 or 2, in which the VCAM-1 ligand is selected from the group consisting of B 2702-84.75/75-84, B 2702.84-75, 4(B 2702.84-75)Raft, and VCAM-1 ligand aptamers.

4. Use of a VCAM-1 ligand according to any one of Claims 1 to 3, in which the VCAM-1 ligand is associated with a marker.

5. Use of a VCAM-1 ligand according to Claim 4, in which the said marker is selected from the group consisting of a radioactive nuclide, a fluorophore, a chemiluminescent compound, a bioluminescent compound and a MRI contrast agent.

6. Use of a VCAM-1 ligand according to Claim 4 or 5, in which the said marker is selected from the group consisting of 123_{I}, ¹²⁵I, ^{99m}Tc, ¹¹¹In, ¹⁸F , ⁶⁷Ga, nanoparticles, gadolinium, fluorescein, Alexa, cyanine, luciferase and alkaline phosphatase.

7. Use of a VCAM-1 ligand according to any one of Claims 1 to 6, in which the VCAM-1 ligand is labelled and selected from the group consisting of [¹²⁵I]-B 2702.84-75/75-84, [¹²⁵I]-B 2702.84-75, [¹²³I]-B 2702.84-75/75-84, [¹²³I] -B 2702.84-75, [^{99m}Tc]-B 2702.84-75/75-84, [^{99m}Tc]-B 2702.84-75, [¹²³I]4(B 2702.84-75)Raft, [¹²⁵I]-4(B 2702.84-75)Raft, [^{99m}Tc]-4(B 2702.84-75)Raft, and VCAM ligand aptamers labelled with iodine 123, iodine 125 or Tc99m.

8. Use of a VCAM-1 ligand according to any one of Claims 1 to 7, in which the contrast agent can be used for the detection of a cardiovascular disease and/or a risk of an acute ischaemic event in a subject likely to exhibit such a disease.

9. Use of a VCAM-1 ligand according to Claim 8, in which an atheromatous plaque is detected.

10. Use of a VCAM-1 ligand according to Claim 9, in which the atheromatous plaque is a vulnerable atheromatous plaque.

11. Use of a VCAM-1 ligand according to Claim 9 or 10, in which the atheromatous plaque is a vulnerable coronary atheromatous plaque.

12. Use of a VCAM-1 ligand according to any one of Claims 8 to 11, in which the said cardiovascular disease is a coronary disease.

13. Use of a VCAR4-1 ligand according to any one of Claims 8 to 12, in which the said risk of an acute ischaemic event is a risk of myocardial infarction.

14. Use of a VCAM-1 ligand according to any one of Claims 8 to 10, in which the atheromatous plaque is a carotid or renal atheromatous plaque, or is located in the region of an artery of the lower limbs.

15. Use of a VCAM-1 ligand according to any one of Claims 8 to 10 or 14, in which the said risk of acute ischaemic event is a risk of renal embolism.

16. Use of a VCAM-1 ligand according to any one of Claims 8 to 10 or 14, in which the said risk of acute ischaemic event is a risk of cerebral vascular accident.

17. Use of a VCAM-1 ligand according to any one of Claims 1 to 7, in which the contrast agent can be used for the therapeutic monitoring of a cardiovascular disease in a subject in which a cardiovascular disease has been diagnosed.

18. Use of a VCAM-1 ligand for the production of a medicament intended for the therapeutic or preventive treatment of a cardiovascular disease, in which the said VCAM-1 ligand is used for treating a coronary or vulnerable aortic atheromatous plaque, the VCAM-1 ligand being selected from the group consisting of the peptide consisting of the sequence YRLAIRLNER (SEQ ID NO: 1) and its derivatives, the peptide consisting of the sequence RENLRIALRY (SEQ ID NO: 5) and its derivatives, the peptide consisting of the sequence YRLAIRLNERRENLRIALRY (SEQ ID NO: 2) and its derivatives, the composition 4 (B2702.84-75)Raft and its derivatives and VCAM-1 ligand aptamers; the derivatives of a peptide and a compound being a peptide or a compound wherein the sequence RENLRIALRY (SEQ ID NO: 5) has been modified by addition, deletion, substitution, or modification of an amino acid, in which the said VCAM-1 ligand is coupled with a cytotoxic agent.

19. Use according to Claim 18, in which the said cytotoxic agent is a radioactive isotope selected from within the group consisting of iodine 131 and yttrium 90 (⁹⁰Yt).

20. Use according to any one of Claims 18 to 19, in which the said VCAM-1 ligand is selected from the group consisting of the peptide B 2702.84-75/75-84, the peptide B 2702.84-75, the compound 4(B 2702.84-75)Raft, and VCAM ligand aptamers.

21. Use according to any one of Claims 18 to 20, in which the said VCAM-1 ligand is used for the prevention of an acute ischaemic event selected from within the group consisting of a myocardial infarction, a cerebral vascular accident, a renal embolism, acute ischaemia of a limb, and a rupture of an aortic aneurism.

22. VCAM-1 ligand for its use for the therapeutic or preventive treatment of a cardiovascular disease, in which the said VCAM-1 ligand is used for treating a vulnerable coronary or aortic atheromatous plaque, the VCAM-1 ligand being selected from the group consisting of the peptide consisting of the sequence YRLAIRLNER (SEQ ID NO: 1) and its derivatives, the peptide consisting of the sequence RENLRIALRY (SEQ ID NO: 5) and its derivatives, the peptide consisting of the sequence YRLAIRLNERRENLRIALRY (SEQ ID NO: 2) and its derivatives, the composition 4 (B2702.84-75)Raft and its derivatives and VCAM-1 ligand aptamers, the derivatives of a peptide and a compound being a peptide or a compound wherein the sequence RENLRIALRY (SEQ ID NO: 5) has been modified by addition, deletion, substitution, or modification of an amino acid,
in which the said VCAM-1 ligand is coupled with a cytotoxic agent.

23. VCAM-1 ligand according to Claim 22, in which the said cytotoxic agent is a radioactive isotope selected from within the group consisting of iodine 131 and yttrium 90 (⁹⁰Yt).

24. VCAM-1 ligand according to any one of Claims 22 to 23, in which the said VCAM-1 ligand is selected from the group consisting of the peptide B 2702.84-75/75-84, the peptide B 2702.84-75, the compound 4(B 2702.84-75)Raft, and VCAM ligand aptamers.

25. VCAM-1 ligand according to any one of Claims 22 to 24, in which the said VCAM-1 ligand is used for the prevention of an acute ischaemic event selected from within the group consisting of a myocardial infarction, a cerebral vascular accident, a renal embolism, acute ischaemia of a limb, and a rupture of an aortic aneurism.

## Patentansprüche

1. Verwendung eines VCAM-1-Liganden als Kontrastmittel in der medizinischen, nicht invasiven *in vivo* Bildgebung, wobei der VCAM-1-Ligand aus der Gruppe ausgewählt wird, die aus dem Peptid bestehend aus der Sequenz YRLAIRLNER (SEQ ID Nr: 1) und seinen Derivaten, dem Peptid bestehend aus der Sequenz RENLRIALRY (SEQ ID Nr. 5) und seinen Derivaten, dem Peptid bestehend aus der Sequenz YRLAIRLNERRENLRIALRY (SEQ ID Nr. 2) und seinen Derivaten, aus der Verbindung 4 (B2702.84-75)Raft und ihren Derivaten, und aus VCAM-1-Liganden-Aptameren besteht, wobei die Derivate der Peptide und der Verbindung ein Peptid oder eine Verbindung sind, bei dem/bei der die Sequenz RENLRIALRY (SEQ ID Nr. 5) durch Hinzufügen, Entfernen, Substitution oder Modifizierung einer Aminosäure modifiziert wurde.

2. Verwendung eines VCAM-1-Liganden gemäß Anspruch 1, wobei die medizinische Bildgebung eine Technik ist, die aus der Gruppe bestehend aus der Szintigraphie, der Magnetresonanztomographie und der optischen Bildgebung ausgewählt wird.

3. Verwendung eines VCAM-1-Liganden gemäß Anspruch 1 oder 2, wobei der VCAM-1-Ligand aus der Gruppe ausgewählt wird, die aus B 2702-84.75/75-84, B 2702.84-75, 4(B 2702.84-75)Raft und aus VCAM-1-Liganden-Aptameren besteht.

4. Verwendung eines VCAM-1-Liganden gemäß irgendeinem der Ansprüche 1 bis 3, wobei der VCAM-1-Ligand mit einem Marker assoziiert ist.

5. Verwendung eines VCAM-1-Liganden gemäß Anspruch 4, wobei der Marker aus der Gruppe bestehend aus einem radioaktiven Nukleid, einem Fluorophor, einer chemolumineszenten Verbindung, einer biolumineszenten Verbindung und einem MRT-Kontrastmittel ausgewählt wird.

6. Verwendung eines VCAM-1-Liganden gemäß Anspruch 4 oder 5, wobei der Marker aus der Gruppe bestehend aus ¹²³I, ¹²⁵I, ^{99m}Tc, ¹¹¹In, ¹⁸F, ⁶⁷Ga, Nanopartikeln, Gadolinium, Fluoreszin, Alexa, Cyanin, Luciferase und alkalischer Phosphatase ausgewählt wird.

7. Verwendung eines VCAM-1-Liganden gemäß irgendeinem der Ansprüche 1 bis 6, wobei der VCAM-1-Ligand markiert wird und aus der Gruppe ausgewählt wird, die aus [¹²⁵I]-B 2702.84-75/75-84, [¹²⁵I]-B 2702.84-75, [¹²³I]-B 2702.84-75/75-84, [¹²³I]-B 2702.84-75, [^{99m}Tc]-B 2702.84-75/75-84, [^{99m}Tc]-B 2702.84-75, [¹²³I]-4 (B 2702.84-75)Raft, [¹²⁵I]-4 (B 2702.84-75)Raft, [^{99m}Tc]-4(B 2702.84-75)Raft, und mit Jod 123, Jod 125 oder mit Tc99m markierten VCAM-Liganden-Aptameren besteht.

8. Verwendung eines VCAM-1-Liganden gemäß irgendeinem der Ansprüche 1 bis 7, wobei das Kontrastmittel für das Nachweisen einer kardiovaskulären Erkrankung und/oder eines Risikos für eine akute ischämische Attacke bei einer Person, die für das Auftreten einer derartigen Erkrankung empfänglich ist, nutzbar ist.

9. Verwendung eines VCAM-1-Liganden gemäß Anspruch 8, wobei ein atheromatöser Plaque nachgewiesen wird.

10. Verwendung eines VCAM-1-Liganden gemäß Anspruch 9, wobei der atheromatöse Plaque ein vulnerabler atheromatöser Plaque ist.

11. Verwendung eines VCAM-1-Liganden gemäß Anspruch 9 oder 10, wobei der atheromatöse Plaque ein vulnerabler koronarer atheromatöser Plaque ist.

12. Verwendung eines VCAM-1-Liganden gemäß irgendeinem der Ansprüche 8 bis 11, wobei die kardiovaskuläre Erkrankung eine koronare Erkrankung ist.

13. Verwendung eines VCAM-1-Liganden gemäß irgendeinem der Ansprüche 8 bis 12, wobei das Risiko für eine akute ischämische Attacke ein Risiko für einen Myokardinfarkt ist.

14. Verwendung eines VCAM-1-Liganden gemäß irgendeinem der Ansprüche 8 bis 10, wobei der atheromatöse Plaque ein atheromatöser Plaque der Carotis, der Nieren oder ein sich auf Höhe einer Arterie der unteren Extremitäten befindender Plaque ist.

15. Verwendung eines VCAM-1-Liganden gemäß irgendeinem der Ansprüche 8 bis 10 oder 14, wobei das Risiko für eine akute ischämische Attacke ein Risiko für eine Nierenembolie ist.

16. Verwendung eines VCAM-1-Liganden gemäß irgendeinem der Ansprüche 8 bis 10 oder 14, wobei das Risiko für eine akute ischämische Attacke ein Risiko für einen Schlaganfall ist.

17. Verwendung eines VCAM-1-Liganden gemäß irgendeinem der Ansprüche 1 bis 7, wobei das Kontrastmittel für das therapeutische Behandeln einer kardiovaskulären Erkrankung bei einer Person verwendbar ist, bei der eine kardiovaskuläre Erkrankung diagnostiziert wurde.

18. Verwendung eines VCAM-1-Liganden zum Herstellen eines Medikaments, das zur therapeutischen oder präventiven Behandlung einer kardiovaskulären Erkrankung bestimmt ist, wobei der VCAM-1-Ligand verwendet wird, um einen vulnerablen koronaren oder Aorten- atheromatösen Plaque zu behandeln, wobei der VCAM-1-Ligand aus der Gruppe ausgewählt wird, die aus dem Peptid bestehend aus der Sequenz YRLAIRLNER (SEQ ID Nr. 1) und dessen Derivaten, dem Peptid bestehend aus der Sequenz RENLRIALRY (SEQ ID Nr. 5) und dessen Derivaten, dem Peptid bestehend aus der Sequenz YRLAIRLNERRENLRIALRY (SEQ ID Nr. 2) und dessen Derivaten, der Verbindung 4(B2702.84-75)Raft und deren Derivaten, und VCAM-1-Liganden-Aptameren besteht, wobei die Derivate der Peptide und der Verbindung ein Peptid oder eine Verbindung sind, bei dem/bei der die Sequenz RENLRIALRY (SEQ ID Nr. 5) durch Hinzufügen, Entfernen, Substitution oder Modifizierung einer Aminosäure modifiziert wurde,
wobei der VCAM-1-Ligand an ein zytotoxisches Mittel gebunden ist.

19. Verwendung gemäß Anspruch 18, wobei das zytotoxische Mittel ein radioaktives Isotop ist, das aus der Gruppe bestehend aus Jod 131 und Yttrium 90 (⁹⁰Yt) ausgewählt wird.

20. Verwendung gemäß irgendeinem der Ansprüche 18 bis 19, wobei der VCAM-1-Ligand aus der Gruppe bestehend aus dem Peptid B 2702.84-75/75-84, dem Peptid B 2702.84-75, der Verbindung 4(B 2702.84-75) Raft und VCAM-Liganden-Aptameren ausgewählt wird.

21. Verwendung gemäß irgendeinem der Ansprüche 18 bis 20, wobei der VCAM-1-Ligand zum Verhindern einer akuten ischämischen Attacke verwendet wird, die aus der Gruppe bestehend aus einem Myokardinfarkt, einem Schlaganfall, einer Nierenembolie, einer akuten Ischämie einer Extremität und einer Aortenaneurysmaruptur ausgewählt ist.

22. VCAM-1-Ligand zu dessen Verwendung für die therapeutische oder präventive Behandlung einer kardiovaskulären Erkrankung, wobei der VCAM-1-Ligand verwendet wird, um einen vulnerablen atheromatösen koronaren oder Aorten-Plaque zu behandeln, wobei der VCAM-1-Ligand aus der Gruppe ausgewählt wird, die aus dem Peptid bestehend aus der Sequenz YRLAIRLNER (SEQ ID Nr. 1) und dessen Derivaten, dem Peptid bestehend aus der Sequenz RENLRIALRY (SEQ ID Nr. 5) und dessen Derivaten, dem Peptid bestehend aus der Sequenz YRLAIRLNERRENLRIALRY (SEQ ID Nr. 2) und dessen Derivaten, der Verbindung 4(B2702.84-75)Raft und deren Derivaten und aus VCAM-1-Liganden-Aptameren besteht, wobei die Derivate der Peptide und der Verbindung ein Peptid oder eine Verbindung sind, bei dem/bei der die Sequenz RENLRIALRY (SEQ ID Nr. 5) durch Hinzufügen, Entfernen, Substitution oder Modifizierung einer Aminosäure modifiziert wurde,
wobei der VCAM-1-Ligand an ein zytotoxisches Mittel gebunden ist.

23. VCAM-1-Ligand gemäß Anspruch 22, wobei das zytotoxische Mittel ein radioaktives Isotop ist, das aus der Gruppe bestehend aus Jod 131 und Yttrium 90 (⁹⁰Yt) ausgewählt wird.

24. VCAM-1-Ligand gemäß irgendeinem der Ansprüche 22 bis 23, wobei der VCAM-1-Ligand aus der Gruppe ausgewählt wird, die aus dem Peptid B 2702.84-75/75-84, dem Peptid B 2702.84-75, der Verbindung 4(B 2702.84-75)Raft und VCAM-Liganden-Aptameren besteht.

25. VCAM-1-Ligand gemäß irgendeinem der Ansprüche 22 bis 24, wobei der VCAM-1-Ligand zum Verhindern einer akuten ischämischen Attacke verwendet wird, die aus der Gruppe bestehend aus einem Myokardinfarkt, einem Schlaganfall, einer Nierenembolie, einer akuten Ischämie einer Extremität und einer Aortenaneurysmaruptur ausgewählt ist.
